# EUROPEAN PATENT APPLICATION

(11) **EP 3 885 354 A1**
(43) Date of publication of application: **29.09.2021**
(21) Application number: 20382218.4
(22) Date of filing: 23.03.2020
(51) Int. Cl.: C07K 14/415, C12N 15/09, C12N 15/82

(54) **LOW INTAKE CESIUM AND PARTHENOCARPY PLANTS**

(71) Applicant: Consejo Superior de Investigaciones Científicas (CSIC), 28006 Madrid (ES)
(72) Inventor: RUBIO MUÑOZ, Francisco, 30100 Murcia (ES); NIEVES CORDONES, Manuel, 30100 Murcia (ES); LARA HURTADO, Alberto, 30100 Murcia (ES); MARTINEZ LOPEZ, Vicente, 30100 Murcia (ES); RÓDENAS CASTILLO, Reyes, 30100 Murcia (ES)
(74) Representative: Pons

(57) **Abstract**

The present invention discloses a potassium (K+) transporter gene (*SIHAK5*) in tomato plants whose silencing mutation in tomato plants leads to low intake of Cesium (Cs+) by the roots and reduced accumulation of Cs+ at the roots and fruits, and therefore protects said plants from accumulating Radiocesium reducing the risk of food products being contaminated with radiation. Furthermore, the silencing mutation results in parthenocarpy plants.

SEQUENCE LISTING

<110> Consejo Superior de Investigaciones Científicas - CSIC

<120> Low intake cesium and parthenocarpy plants

<130> EP1641.1527

<160> 3

<170> BiSSAP 1.3.6

<210> 1
<211> 51
<212> PRT
<213> Solanum lycopersicum


<400> 1
Met Glu Ser Thr Lys Ser Glu Glu Glu Val Asn Val Gly Gln Gln Gln
1               5                   10                  15
Leu Lys Asp Arg Lys Val Ser Trp Ala Lys Leu Gly Arg Val Asp Ser
            20                  25                  30
Leu Asn Met Glu Ala Gly Lys Val Ser Ser Thr Gln Ser Lys Thr Trp
        35                  40                  45
Leu Gln Gly
    50

<210> 2
<211> 2361
<212> DNA
<213> Solanum lycopersicum


<400> 2
atggagagca caaaaagtga agaggaagtt aatgtagggc agcaacaact caaagatagg      60

aaagtatcat gggcaaagct aggtcgtgtt gattcactca acatggaagc tggaaaagtt     120

tcttccactc aagcaagaca tggctccaag ggtgattgga agacaatatt gagtttagca     180

tttcaatcag taggagttat ctatggagac atagggactt ctcctctcta tgtttttgca     240

agtactttta ctgatgaaat taaacacaag gatgacattc taggtgtatt gtccctcatc     300

atttatacca taatgcttgt acctatgact aagtatgtct tcattgtctt atgggccaac     360

gacaatggcg atgggggagc atttgcattg tattccttat tgtgtagata tgcaaaagtg     420

agtcttatcc caaatcaaca gccagaggac agagaacttt cacactatag tttggactta     480

ccatccaatc atattaaaag agctcaaagg atcagacagg gtttggagaa gagcaagttt     540

gcaaaaatat ttcttgtatt tcttgccatt cttggaacat ctatggtcat tggtgatgga     600

gttctcacac cctgtatttc agtgctctct gccgtaagtg gaataaagcc cctaggccaa     660

gacgctatta tgggtatttc catagcaata ttggtgatcc ttttcagtct tcaacgtatg     720

ggtacagaca aggttggata tacatttgct ccagccatat gtgtttggtt tttattcata     780

agtggcattg gtctctacaa cttgttcaag tatgatgtga ccgtgttacg cgcctttaat     840

ccaatgtaca tcattcatta cttcaaaaga aacggtaaaa aaggatggat ttctcttggc 900

ggagttttcc tttgcatcac aggatctgaa gctatgtttg ctgatttagg tcacttcagt 960

gtgcgttcta ttcaaataag cttcagttgt ctcgtatttc cagctttact ctcagcttac 1020

agtggccaag ctgcttatct cacaaaattc cctgaaaatg tggcgaacac cttctatgac 1080

tgtattccag gtccacttta ctggccaaca tttgtagtgg ctgttgctgc agcaattatt 1140

gcaagtcaag cgatgatttc tggaactttt tcaattgttg ctcaagcaca aaacgtaggt 1200

tgttttccaa gggtgaaggt aattcacaca tcaaccaaac atgacggtca agtatacatc 1260

ccggaactca attacttcct catgattgct tgtgttttag ttactcttag cttcaaaaca 1320

accgaaaaac tgggacatgc ttatggtatt gctgtcgtta gtgctgaaat cataacgaca 1380

catatggtaa cattagttat gcttgttata tggaagacga aaatatggtg gattacctta 1440

ttctacgcgg tgtaccttag tattgaatct acctatttct ctgctcagct tactaaattc 1500

actcaaggtg gttatctacc catggcattc tcagttgtgc ttgtgatcat tatgggaact 1560

tggtattatg ttcaaaaact acgatacgaa tttgagctca acaacaaggt ttcgactgag 1620

tacattagtg atttggctaa taatccagac atcaaaagag taccaggtat aggattacta 1680

tactctgaat tagtacaggg gattccacct attttccctc attttgtctc caatattccc 1740

tctgttcact ctgttatcgt tctcgtgtca atcaaatcaa tacctataag taaagtagca 1800

ctacaagaac gtttcttgtt ccgtcatgtt gaacctagag agtacaaggt attcagatgt 1860

gtagtcagat taggctacaa ggatcaactt ggtgacacaa tggattttga aaaccaatta 1920

gttgaacaac taaacaaatt catacgacac gaacattaca ttcttgaagc acatgaacaa 1980

gtggtgaata gagagaaaac atccagggtt cacatagagg aagagatgga acaaccacaa 2040

ccacaacaac aagtggattc tacaacatca ccatcaacgc gttccattca atctaatcgt 2100

tcatcgagta gaatacaagt gttgcatcca aatgcttcag gacaagaaga gacacaattt 2160

atcgaaaaag ctaaggatca aggagtgttc tatctattgg cagaagcaga ggtgattgct 2220

aaacaagatt cgtcgtttgt gaagaaaggt attatcaatt atggttatag tttcttgcgc 2280

aagaatttca ggcaagggga gaaagttatg gcaattcctc agacaaggct tctaagggtt 2340

ggcatgactt atgagctata a 2361

<210> 3
<211> 20
<212> DNA
<213> Artificial Sequence

<220>
<223> sgRNA

<400> 3

ggagccatgt cttgcttgag                                                20

## Description

The invention relates to the silencing of a K+ transporter gene in agricultural important plants. The mutation of the transporter leads to low intake of cesium and generation of seedless fruits by the transgenic plants.

### BACKGROUND ART

Nuclides released after nuclear accidents have become an important threat for agriculture due to the large area of land contaminated and its persistence in time (decades). Examples of such releases are the nuclear accidents of Chernobyl and Fukushima. Radiocesium, one of the emitted nuclides, has relatively long half-lives (2.06 years for ¹³⁴Cs, 30.2 years for ¹³⁷Cs), and contamination of the environment and foods with radiocesium has been and will be of concern for a long time. Radiocesium deposited in the soil sticks to soil particles, however a small portion is redistributed into a soil solution that is readily absorbed by plants. Uptake and transport of cesium (Cs+) by plants is an important determinant of the degree of radiocesium contamination of foods. Thus, the consumption of radiocesium containing food by cattle and humans imposes serious health concerns.

It is known that potassium (K+) and Cs+ are taken up by roots in a similar fashion and root K+ uptake systems may constitute the gate for Cs+ accumulation into the plant. K+ is taken up from the soil solution by the roots through specific transport systems located at the plasma membrane of epidermal and cortical root cells. Research in the model plant *Arabidopsis thaliana* has allowed the characterization of the main systems involved in root K+ uptake and their regulatory mechanisms (Alemán et al., 2011 Plant and Cell Physiology 52(9): 1603-1612). The AtHAK5 transporter is the only system mediating K+ uptake at concentrations lower than 20 µM. At higher concentrations, from 20 to 200 µM, both AtHAK5 and the AKT1 channel contribute to uptake. At concentrations higher than 500 µM K+, AtHAK5 contribution is very low and AKT1 becomes the predominant pathway for K+ uptake. Thus, this type of transporters and channels are interesting targets for crop biotechnology. Cesium uptake by plants has no known benefice for plants but leads to growth impairment mostly due to a reduction of influx of K+ (Adams et al., 2019 Plant Cell Physiol 60 (1), 63-76).

Biotechnological improvements to crop plants that attempt to reduce the risk of accidental contamination of food by harmful agents to human and animals have long been present in agricultural industry. Such improvements normally target ion transporters in order to reduce the intake of said harmful agents. As examples, the document WO2013065517 discloses transporter genes and transporter proteins that control the intake of cadmium absorption by roots in rice cultivars and rice mutants that have low cadmium intake. The document WO2005087928 discloses transporter genes capable of impart boron-tolerance in *A. thaliana.*

Since the Fukushima Daiichi nuclear power plant accident in March 2011 there was an increase on the work and research into radioactive decontamination or prevention of contamination. The document JP2013158327 discloses a Cs+ transporter protein as well as the gene that encodes such protein and a rice plant which produces said protein. Likewise, the document JP2018033326 also discloses a gene that controls cesium absorption and a low-absorptive rice plant. The document JP2013217820 discloses a method to decontaminate biological materials like leaves, straw, weeds, plant residues, wood chips and the like, from Cs+ by the addition of filamentous fungi to an aqueous solution containing the contaminated materials.

Despite such works, most of the inventions disclosed are related to the crop rice and have limited application in other plants, which are also of commercial interest in the agricultural industry. Therefore, other agricultural products, specially products commercially important, with low intake of Cs are necessary in order to reduce the possibility of contamination of the food chain in areas affected by contaminated soils.

### DESCRIPTION OF THE INVENTION

The present invention discloses a potassium (K+) transporter gene in tomato plants (*SIHAK5*) whose silencing mutation only affects the intake of K+ in situations of K+ starvation, but leads to low intake of Cesium (Cs+) by the roots independently of the K+ status, and therefore protects said plants from accumulating Radiocesium, reducing the risk of food products being contaminated with radiation. Furthermore, the plants showed low accumulation of Cs+ both in the root as well as in the fruits, an essential aspect to allow use and commercialization of plants grown in contaminated agricultural lands.

Additionally, the mutation of the K+ transporter gene has the unexpected effect of leading to parthenocarpic plants where the number of seeds in the fruits is greatly reduced or totally absent. Such plants are of high interest commercially since seedless fruits are tastier and have longer shelf-life than seeded fruits. Furthermore, seedless tomatoes are also important for the production of tomato sauce, since the seeds hinder the process of tomato juice extraction.

The transporter gene mutation herein described creates an early stop codon which leads to a truncated protein without function. Therefore, such mutation is a gene silencing mutation or knockout (KO) mutation, which leads to a loss of function of the gene or the protein encoded by the gene.

Therefore, a first aspect of the present invention relates to a transgenic plant (from here onwards the plant of the invention), to a reproductive or propagating plant material (from here onwards the reproductive or propagating plant material of the invention), or to a cultured plant cell (from here onwards the cultured plant cell of the invention) characterized in that it comprises a gene (from here onwards the gene of the invention) with at least 90%, 95% or 100% sequence identity with SEQ ID NO: 2 wherein said gene has been silenced.

The term "plant" as used herein includes whole plants, any reproductive or propagating material for a plant, progeny of the plants and parts of plants, including seeds, siliques, fruits, leaves, flowers, shoots, stems, roots, isolated plant cells, tissues and organs. References to a plant may also include plant cells, plant protoplasts, plant tissue cultures, plant calluses, plant clusters and plant cells which are intact in plants or parts of plants, such as embryos, pollen, ovules, seeds, leaves, flowers, branches, fruits, grains, spikes, ears, hulls, stems, roots, root tips and the like. The offspring, variants and mutants of any of the transgenic plants described herein are within the scope of the present invention. The seeds of any of said transgenic plants are also included.

The term "plant cell" as used herein includes plant cells derived and/or isolated from plant cell tissue or from plant cell culture. As it is used herein, the term "parts of a plant" includes any part or parts of a plant including the seeds, siliques, fruits, leaves, flowers, shoots, stems and / or roots.

The term "transgenic", or its synonym "transformed", as used herein refer to a host organism such as a plant cell, plant or part of a plant in which an exogenous nucleic acid molecule has been introduced or an endogenous nucleic acid has been genetically engineered to be distinct from the naturally present endogenous nucleic acid. It is understood that transformed plant cells, plants or parts of plants cover not only the final product of a transformation process, but also the transgenic progeny thereof. The latter or nth-generation plants can be produced by sexual reproduction, i.e. fertilization. Furthermore, the plants can be a gametophyte (haploid stage) or a sporophyte (diploid stage).

The term "exogenous" when used herein refers to a nucleic acid molecule (for example, a DNA sequence), gene or protein, which originates from a particular source that is foreign to the cell, plant or part of a plant in which it is introduced. The exogenous nucleic acid molecule can be introduced in the plant in a stable or transient manner to produce an RNA molecule and/or a polypeptide molecule. An "endogenous" nucleic acid molecule, gene or protein of, is a nucleic acid molecule (for example, a DNA sequence), gene or protein associated in a natural manner with, or native of, a plant cell, plant or part of a plant.

The term "gene" is widely used to refer to any segment of DNA associated with a biological function. Therefore, the genes include encoding sequences and/or the regulating sequences required for their expression. The genes also include unexpressed DNA segments which, for example, form recognition sequences for other proteins. The genes can be obtained from a variety of sources, including cloning from a source of interest or synthesis from known or predicted sequence information, and may include sequences designed to have the desired parameters.

For purposes of the present invention, the term "transgenic plant" refers to plants whose genetic material has been deliberately modified as to alter the gene *SIHAK5,* which comprises SEQ ID NO: 2, or a gene with at least 90%, 95% or 100% sequence identity to the SEQ ID NO: 2, or a gene which consists of SEQ ID NO: 2. Specifically, the transgenic plants of the invention have been modified by genetic engineering to silence the expression of the gene *SIHAK5* described herein and/or to modify its expression product in order for said product to be non-functional.

The term "silenced" as used herein refers to decreasing, reducing, or inhibiting the expression of the target gene or target allele by at least about 50%, 60%, 70%, 75%, 80%, 85% to 100% compared to its normal expression in a wild type plant. In addition, the term "silenced" is also used herein as a synonym of "knockout" which refers to the modification of the gene nucleotide sequence in a way as to produce a non-functional messenger RNA or non-functional protein. In a preferred embodiment the plant of the invention, or the reproductive or propagating plant material of the invention, or the cultured plant cell of the invention is characterized in that the gene silenced has at least 90, 91, 92, 93, 94, 95, 96, 97, 98, 99, or 100% identity with the gene of the invention.

The term "wild type" (also written "wildtype", "wild-type" or WT), as used herein, refers to a typical form of a plant or a gene as it most commonly occurs in nature. A "wild type plant" refers to a plant with the most common phenotype of such plant in the natural population.

The term "identity" as used herein refers to the proportion of identical amino acids or nucleotides between two compared peptides/proteins or nucleotide sequences, respectively, along their full-length sequence. The methods for comparing sequences are known in the state of the art, and include, but not limited to, the programs BLASTP or BLASTN, ClustalW and FASTA. We can consider that peptides, proteins or nucleotide sequences with percent identities of at least 90% will maintain the same properties as the sequence to which they refer.

In a preferred embodiment of the plant of the invention, or the reproductive or propagating plant material of the invention, or the cultured plant cell of the invention characterized in that it comprises the polypeptide according to SEQ ID NO: 1.

The terms "peptide", "polypeptide" and "protein" are used herein interchangeably to describe a series of at least two amino acids covalently linked by peptide bonds or modified peptide bonds such as isosteres. No limitation is placed on the maximum number of amino acids which may comprise a peptide or protein. Furthermore, the term polypeptide extends to fragments, analogues and derivatives of a peptide, wherein said fragment, analogue or derivative retains the same biological functional activity as the peptide from which the fragment, derivative or analogue is derived.

The term "nucleotide" refers to the basic building blocks of nucleic acids comprising or consisting of a nucleobase bound, ribose or deoxyribose and at least one phosphate group. The basic building blocks are cytosine (C), guanine (G), adenine (A), thymine (T), and uracil (U).

In the present invention the polypeptide according to SEQ ID NO: 1 relates to a non-functional protein.

In another preferred embodiment of the plant of the invention, or the reproductive or propagating plant material of the invention, or the cultured plant cell of the invention belongs to the *Solanum sp.* genus or the *Capsicum sp.* genus. In a yet more preferred embodiment, the species are selected from the list consisting of: *S. lycopersicum, S. tuberosum, S. pennellii, S. pimpinellifolium, S. peruvianum, S. cheesmanii, S. galapagense, S. chilense, S. melongena, S. aethiopicum, S. quitoense, S. torvum, S. muricatum, S. betaceum* and *Capsicum annuum.* It is to be understood that a species also includes all subspecies and varieties or cultivars therein.

The term "cultivar" as used herein refers to a plant having a biological status other than a "wild" status, which "wild" status indicates the original non-cultivated, or natural state of a plant or accession. The term "cultivar" includes, but is not limited to, semi-natural, semi-wild, weedy, traditional cultivar, landrace, breeding material, research material, breeder's line, synthetic population, hybrid, founder stock/base population, inbred line (parent of hybrid cultivar), segregating population, mutant/genetic stock, and advanced/improved cultivar.

The term "variety" as used herein refers to a group of plants within a species that share constant characters that separate them from the typical form and from other possible varieties within that species. While possessing at least one distinctive trait, a variety is also characterized by some variation between individuals within the variety, based primarily on the Mendelian segregation of traits among the progeny of succeeding generations.

Therefore, in a yet more preferred embodiment, the species *S. lycopersicum* includes, without limitation, the varieties/cultivars Baladre, Bella Rosa, Cabri, Caracas, Carbon, Cherry, Dombito, Estrella, Juboline, Kumato, Marglobe, Meltine, Monserrat, Muchamiel, Nemato, Pera de Girona, RAF, Roma, Sobeto, Sonatine, Terrades, Vergel, Adoration, Alicante, Azoychka, Better Boy, Big Beef, Big Rainbow, Blaby Special, Black Krim, Brandywine, Campari, Celebrity, Cherokee Purple, Canario (tomato), Tomkin, Early Girl, Enchantment, Ferris Wheel, Flamenco, Fourth of July, Garden Peach, Gardener's Delight, Granadero, Great White, Green Zebra, Hanover tomato, Japanese Black Trifele, Jubilee, Juliet, Kumato, Lillian's Yellow, Matt's Wild Cherry, Micro-Tom, Moneymaker, Monterosa, Mortgage Lifter, Mr. Stripey, Pantano Romanesco, Plum tomato, Raf tomato, Rebellion, Red Currant, Rosa de Barbastro, San Marzano, San Pedro, Sasha Altai, Tiny Tim, Cherry Bambelo, Cherry Nebula, Santorini, Tomaccio, Yellow Pear and White Queen.

In another preferred embodiment, the species *S. tuberosum* includes, without limitation, the varieties/cultivars Kennebec, monalisa, Desirée, bintje, Álava, Palogan, Pedro Muñoz, Roja Riñón, Duquesa, Goya, Olalla, Turia, Victor, Lora, Gauna, Alda, Belda, Buesa, Iturrieta, Diba, Fénix, Onda, Arene, Asun, Ayala, Edurne, Gorbea, Idoia, Iker, Inca, Isla, Mayka, Mikel, Montico, Nagore, Nerea, Zadorra, Zarina and Zela.

In another preferred embodiment, the species *S. melongena* includes, without limitation, the varieties/cultivars De Murica, Baffa, Bonita Vittoria, Palermitana, Azucarada, Sabelle RZ F1, Black Beauty, Redonda common from Florencia, Redonda Negra, Redonda Bianca, Próspera, Lety, Durona Copa Negra, Ovalada Tipología Maya, Seda Violeta Larga, Blanca Oval, New York Giant, Galine, Jersey King, Giotto, Violeta de Nápoles, Violeta larga Palermitana, Violeta Enana Precoz, Riminese, Larga Negra, Morella, Mary, Ideal, Mirabelle, Perlita and Barbentane.

In a preferred embodiment, the species *Capsicum annuum* includes, without limitation, the varieties/cultivars Anaheim, Aleppo, Anaheim, Banana, Bird's Eye, Black Heart, Black Hungarian, Cascabel, Cayenne (Red), Cherry, Cheongyang, Chilaca, Chiltepin, Chimayó, Cubanelle, Dangjo, De Árbol, Facing Heaven, Fish, Fresno, Friggitelli (Peperoncini), Guntur Sannam, Hungarian Wax, Italian Sweet, Jalapeño, Korean chili, Medusa, Mirasol, NuMex peppers, Peter Pepper, Peperoncino, Pequin, Piment d'Espelette, Padrón, Poblano, Prik Kee Nu, Puya, Santa Fe Grande, Serrano, Shishito.

In another preferred embodiment of the plant of the invention, or the reproductive or propagating plant material of the invention, or the cultured plant cell of the invention are characterized in that have low intake of Cs+ compared to wild-type control plants, preferably Radiocesium.

The statement "low intake of Cesium compared to wild-type control plants" refers to a reduction in the level of absorption of Cs+ by the plants, specifically by the plants roots and/or fruits or other plant parts, of at least 30 to 100%, preferably of 40, 50, 60, 70 ,80, 85, 90, 95 or 100% compared to the level of absorption of a wild type plant, specifically by the plants roots and/or fruits or other plant parts, in the same culture conditions.

The term Radiocesium as used herein refers to the isotopes of Cs+, particularly longer half-life isotopes, specifically the isotopes with mass numbers of 129, 131, 132, 134, 135, 136 and 137, preferably the isotopes ¹³⁴Cs and/or ¹³⁷Cs.

In another preferred embodiment of the plant of the invention, or the reproductive or propagating plant material of the invention, or the cultured plant cell of the invention are characterized in that it is parthenocarpy.

As used herein, the term "parthenocarpy" refers to production of seedless fruits without pollination and fertilization in a plant, exhibiting enlargement of ovary, receptacles or the like without seed formation. In the context of the present invention, the term "parthenocarpic" refers to the property and the ability of a plant to cause parthenocarpy without the need for artificial parthenocarpic induction treatment such as plant hormone treatment or a certain physical stimulation, respectively.

Another aspect of the present invention refers to a cell, silique, seed, progeny or part of the transgenic plant of the invention (from here onwards the cell, silique, progeny or part of the transgenic plant of the invention), characterized in that it comprises a gene with at least 90%, 95% or 100% sequence identity with SEQ ID NO: 2 wherein said gene has been silenced. In a preferred embodiment the cell, silique, progeny or part of the transgenic plant of the invention are characterized in that the gene silenced comprises the polypeptide according to SEQ ID NO: 1. In another preferred embodiment the cell, silique, progeny or part of the transgenic plant of the invention wherein the parts of the plant are selected from the list consisting of: a leaf, a stem, a flower, an ovary, a fruit or a callus.

Another aspect of the present invention relates to a method for producing the transgenic plant of the invention, or the reproductive or propagating plant material of the invention, or the cultured plant cell of the invention, or a cell, silique, seed, progeny or part of a transgenic plant of the invention said method (from here onwards the method of the invention) comprising the silencing of a gene with at least 90%, 95% or 100% sequence identity with SEQ ID NO: 2. In a preferred embodiment of the method of the invention the transgenic plant, or the reproductive or propagating plant material of the invention, or the cultured plant cell of the invention, or the cell, siliques, seeds, progeny or part of the transgenic plant belong to the genus *Solanum sp.* or the genus *Capsicum sp..* In a more preferred embodiment of the method of the invention the transgenic plant, or the reproductive or propagating material of the invention, or the cultured plant cell of the invention, or the cells, siliques, progeny or part of the transgenic plant belong to a species selected from a list consisting of: *S. lycopersicum, S. tuberosum, S. pennellii, S. pimpinellifolium, S. peruvianum, S. cheesmanii, S. galapagense, S. chilense, S. melongena, S. aethiopicum, S. quitoense, S. torvum, S. muricatum, S. betaceum* and *Capsicum annuum.* It is to be understood that a species includes all subspecies and varieties or cultivars therein. Examples of varieties/cultivars for several species, without limitation, are listed earlier in the description and such varieties/cultivars are valid for the current aspect and its embodiments.

Methods to obtained transgenic plants, reproductive or propagating material, cultured plant cells, cells, siliques, progenies or part of the transgenic plants, specifically wherein genes are silenced, are widely known in the art and an expert would be able to discern the best method to apply to obtain the desired transgenic plant. Such methods include both directed and random mutagenesis strategies. Directed strategies include, but are not limited to, homologous recombination-dependent gene targeting, antisense RNA, directed transposon insertion, virus induced gene silencing, CRISPR techniques. Random mutagenesis strategies are mostly based, but not limited to, in techniques which induce mutations in the DNA of the cells, such as contact with a mutagenic agent, such as a chemical substance (such as ethylmethylsulfonate (EMS), ethylnitrosourea (ENU), etc.) or ionizing radiation (neutrons (such as in fast neutron mutagenesis, etc.), alpha rays, gamma rays (such as that supplied by a Cobalt 60 source), X-rays, UV-radiation, etc.), or a combination of two or more of these.

Another aspect of the present invention relates to a use of the transgenic plant of the invention, or the reproductive or propagating material of the invention, or the cultured plant cell of the invention, or the cell, silique, progeny or part of the transgenic plant of the invention for producing an agro-industrial product, preferably wherein the agro-industrial product is a food or a feed.

Another aspect of the present invention relates to an agro-industrial product production method, preferably wherein the agro-industrial product is a food or a feed, comprising:
a) culturing the transgenic plant of the invention, the reproductive or propagating plant material of the invention, the transgenic plant cell of the invention, or cell, silique, progeny or part of the transgenic plant of the invention, and
b) preparing the agro-industrial product.

Another aspect of the present invention relates to a method for producing a transgenic plant with low intake of Cesium compared to a wild-type control plant, preferably wherein the Cesium is Radiocesium, and that is parthenocarpy, said method comprising culturing the transgenic plant of the invention, reproductive or propagating plant material of the invention, the plant cell of the invention, or cell, silique, progeny or part of the transgenic plant of the invention in a culturing medium comprising a concentration of potassium of more than 100 µM.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

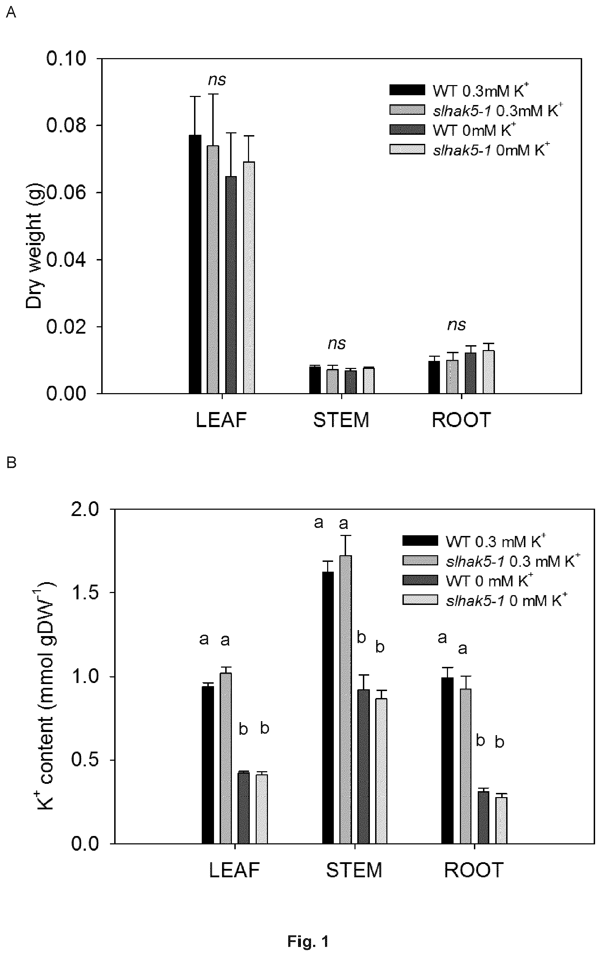

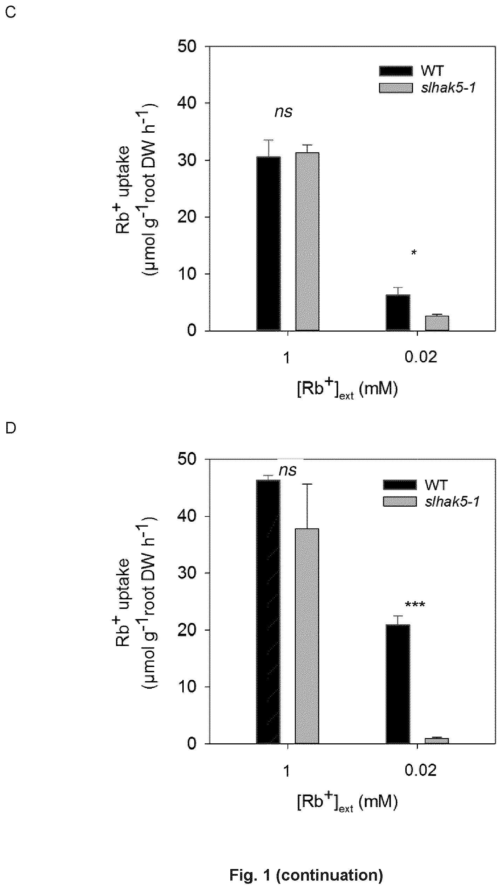

**Fig. 1****. Organ dry weight, K+ concentration and high-affinity K+ uptake in K+-starved WT and *slhak5-1* plants.** Plants of the WT and *slhak5-1* lines were grown for 14 d in complete Hoagland solution containing 0.3 mM K+. Then, a set of plants were grown for 7 d in solution with no K+ and another set remained in the complete solution. After this growth period, plants were transferred to solution with no added K+ and 0.02 mM or 1 mM RbCI (as indicated) for 6 h and then separated in roots, stems and leaves, dried. 4 d later their dry weight was determined. After acid digestion, the internal K+ and Rb+ concentration in tissues were determined. (a) Dry weight of leaves, stems and roots. (b) K+ concentration of leaves, stems and roots. (c and d) Rb+ uptake rates of in K+-sufficient (c) and K+-starved (d) plants at 1 and 0.02 mM Rb+. Shown are mean values (n=5) ± standard error, and bars with different letters are significantly different at P < 0.05 according to Tukey's test. ns denotes not significant. * and *** indicate P < 0.05 and P < 0.001 in Student's t-test, respectively.

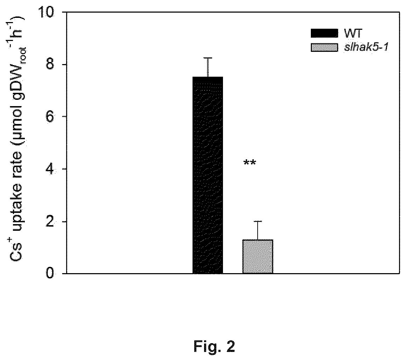

**Fig. 2****. Cs+ uptake rates from a 0.02 mM external Cs+ solution in K+-starved plants.** Plants of the WT and *slhak5-1* lines were grown and starved of K+ as indicated in Fig. 1. Then, plants were transferred to a solution with no added K+ in the presence of 0.02 mM CsCI for 6 h. After this, plant material was harvested and processed to determine the rates of Cs+ uptake. Shown are mean values ± standard error and ** indicates *P* < 0.01 in Student's t-test.

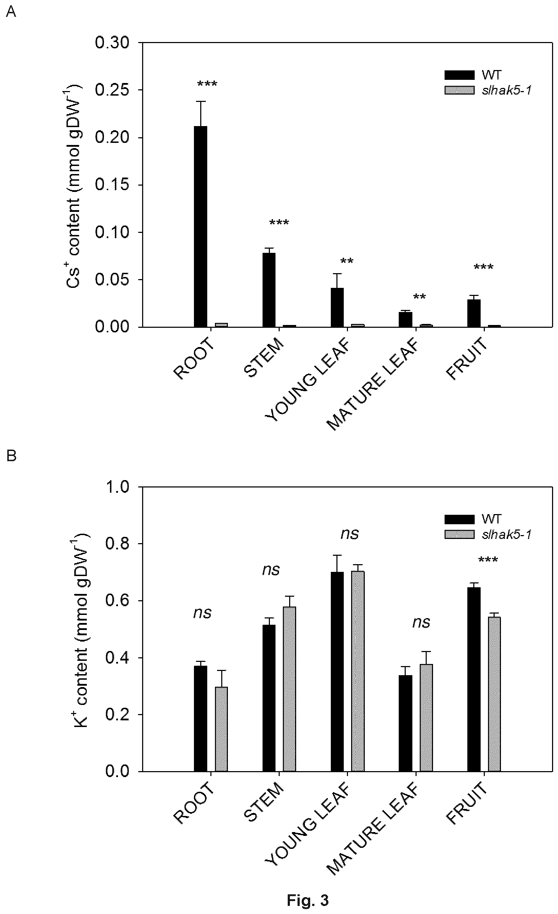

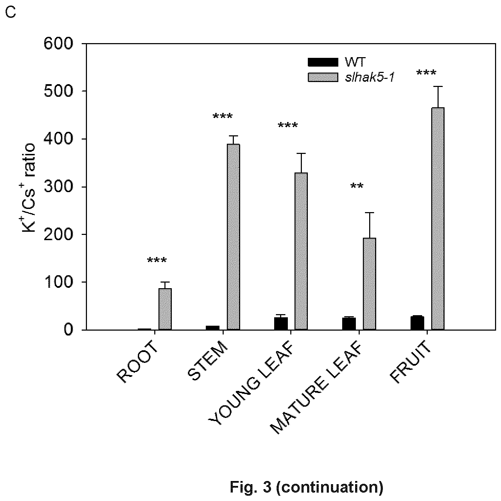

**Fig. 3****. Cs+ and K+ concentrations and K+/Cs+ ratios in organs of plants grown in the presence of 0.3 mM K+ and 0.02 mM Cs+.** Plants of the WT and *slhak5-1* lines were grown in the presence of 1.4 mM K+ for 7 d and then transferred to a solution containing 0.3 mM K+ and 0.02 mM CsCI. Plants were grown for 90 d to allow fructification and fruit harvests. After this, organs were harvested and processed to determine their internal (a) Cs+ and (b) K+ concentrations. (c) The internal K+/Cs+ ratios were calculated. Shown are mean values (n=3) ± standard error. ** and *** indicate P < 0.01 and P < 0.001, respectively, in Student's t-test. ns denotes not significant.

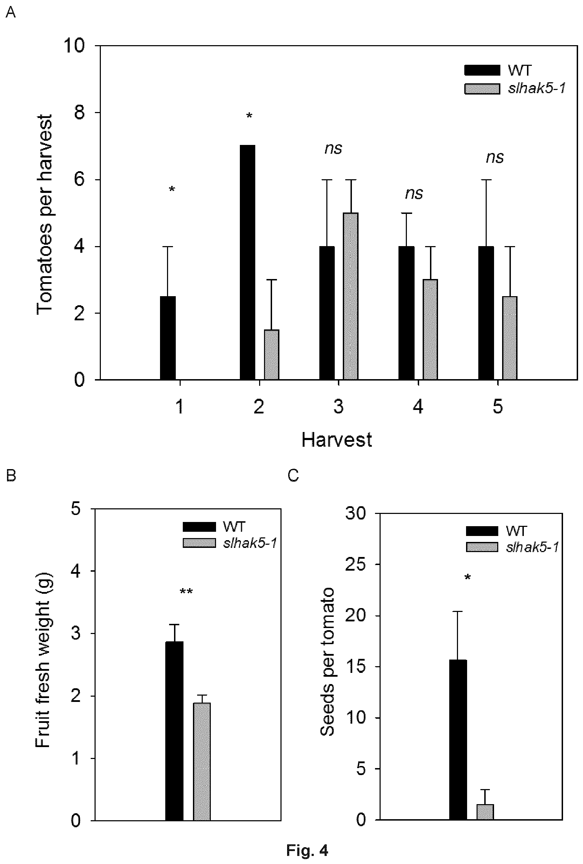

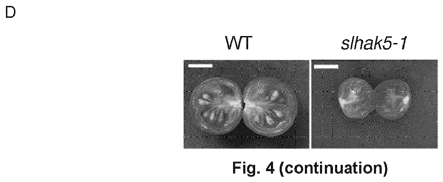

**Fig. 4****. Number of tomato fruits harvested, plant yield and seeds per tomato of WT and *slhak5* plants.** Plants of the WT and *slhak5-1* lines were grown in the presence of 1.4 mM K+ until fructification. Tomato fruits were harvested at different time points. (a) Number of tomato fruits per harvest (1, 2, 3, 4 and 5 denote 90, 97, 106, 118 and 129 d after germination) of three plants for each line. (b) Average fruit fresh weight of WT and *slhak5-1* plants. (c) Average number of seeds per tomato in WT and slhak5-1 plants. Shown are mean values ± standard error and * indicates P < 0.05 in Student's t-test. ns denotes not significant. n=3 for plant tissues and n=15 for fruits. (d) Photographs of WT and *slhak5-1* tomatoes. Scale bar = 1 cm.

### Examples

### Example 1 - Materials and methods

### Plant material and growth conditions

Tomato plants (*Solanum lycopersicum* L. var Micro-Tom) were used throughout the work. Plants were grown as described previously (Nieves-Cordones et al., 2007). Seeds were germinated in a 0.5 mM aerated CSO4 solution for 72 h and then transferred to vermiculite. After 7 d, seedlings were transferred to 8 L containers with aerated nutrient solution that consisted of macronutrients (mM): 1.4 Ca(NO3)2, 0.2 MgSO4 and 0.1 Ca(H2PO4)2 and micronutrients (µM): 50 CaCI2, 12.5 H3BO3, 1 MnSO4, 1 ZnSO4, 0.5 CuSO4, 0.1 H2MoO4, 0.1 NiSO4 and 10 Fe-EDDHA. KCI, NaCl or CsCI were added as indicated in each experiment. Plants were grown in that solution for the indicated time period as indicated in each experiment in a controlled-environment growth chamber with 16/8 h light/night photoperiod, at 25 °C (day) 20 °C (night) temperature, 65 % relative humidity and 360 µmol m⁻² s⁻¹ flux density. pH of the nutrient solutions was adjusted daily to 5.5. Fresh solution was supplied once a week.

### Short term Rb+ and Cs+ uptake experiments, ion content and fruit transfer factor (TFfruit) determinations

Plants were grown as described in each experiment. For short-term Rb+ and Cs+ uptake experiments, plants starved of K+ for 7 d were transferred to K+-free solutions supplemented with 0.02 mM and 1mM of RbCI or CsCI for 6 h. Rb+ or Cs+ uptake rates were calculated from their accumulation in the plant per unit of time and unit of root dry weight. In all experiments, plant organs were harvested, their fresh weight determined and dried in a 65 °C oven for 4 d to determine their dry weight. Plant material was digested with HNO3:HClO4 (2:1, v:v) and K+, Na+ and Rb+ concentration was determined by ICP spectrometry analysis (Iris Interpid II, Thermo Electron Corporation, Franklin, USA). Tissue Cs+ concentration was determined by atomic emission spectrometry in an AAnalist Perking-Elmer 400 spectrometer.

Fruit transfer factor (TFfruit) was calculated as the ratio between fruit Cs+ concentration (expressed in mM) and the external Cs+ concentration (expressed in mM).

### Statistical analysis

Analysis of variance was performed with the Statistix V.8 software for Windows (Analytical Software, Tallahassee, FL). The differences in means were compared by using a Tukey's multiple range test (P < 0.05). Sigma Plot 9.0 was used for data fitting.

### Example 2 - Generation of the slhak5 knockout (KO) mutants

The *slhak5 KO* mutant, the transgenic plant of the invention, was obtained with CRISPR techniques adapted for tomato (Swinnen et al. 2020 Methods Mol Biol. 2083:321-341). A sgRNA spacer sequence 5' GGAGCCATGTCTTGCTTGAG 3' (SEQ ID NO: 3) was cloned in CRISPR plasmids (Zhao et al. 2019, Physiol Plant 167(4):556-563; Ueta et al. 2017, Sci Rep 7(1):507; Yu et al. 2017, Sci Rep 7: 11874; Pan et al. 2016, Sci Rep 6:24765; Fauser et al. 2014, Plant J, 79(2):348-359) to effectively knock-out *SIHAK5* gene in tomato plants. Such sgRNA spacer sequence directs a sgRNA-SpCas9 complex to exon 1 of the *SIHAK5* gene to induce double strand breaks (DSB) in that exon. The mutations generated were screened by sequencing to obtain the *slhak5* knock-out mutant.

### Example 3 - slhak5 plants are affected in root high-affinity K+ uptake

In a first series of experiments we studied the effect of the *KO slhak5* mutation on root K+ uptake. Rubidium (Rb+) was used as a tracer for K+ in uptake experiments at two external concentrations, 1 and 0.02 mM Rb+ in K+-sufficient and -starved plants. After growing *slhak5-1* plants under control Hoagland solution containing 0.3 mM K+ for 14 d, a set of plants were subjected to K+ starvation by growing them for 7 d in the absence of K+. Another set remained in the complete solution with 0.3 mM K+. Then, plants were incubated for 6 h in nutrient solution with no K+, supplemented with 1 or 0.02 mM Rb+. The dry weights and internal K+ concentrations of these plants were determined at the end of the experiment. No differences in the dry weights of the different organs were observed between WT and *slhak5-1* plants (Fig. 1A). As expected, the concentrations of the K+-sufficient organs were higher than those of the K+-starved ones (Fig. 1B). The 7 d K+-starvation treatment reduced organ K+ concentration around 50% and no differences were observed in K+ concentrations between WT and *slhak5-1* organs (Fig. 1B). Rb+ uptake rates were calculated from the Rb+ accumulation within the plant. It could be observed that WT and *slhak5-1* plants showed similar rates of Rb+ uptake in K+-sufficient plants assayed in the presence of 1 mM Rb+ (Fig. 1C). When these K+-sufficient plants were assayed at 0.02 mM Rb+, lower rates of Rb+ uptake than when assayed at 1 mM Rb+ were observed in both lines. Importantly, the uptake rates of the *slhak5-1* line were of a lower magnitude than those of the WT plants (Fig. 1C). K+ starvation increased the rates of Rb+ uptake in WT plants from an external concentration of 1 mM Rb+ and specially from 0.02 mM external Rb+ (Fig. 1D). These results indicated that K+ starvation induced the high-affinity component of K+ uptake. In the presence of 1 mM Rb+, K+-starved *slhak5-1* plants showed a value of Rb+ uptake rate close to that of WT plants (Fig. 1D). However, at 0.02 mM external Rb+, the slhak5-1 line did not show the observed increase of Rb+ uptake displayed by K+-starved WT plants. Indeed, under these conditions, the *slhak5-1* line showed a 22-fold lower rate of Rb+ uptake than WT plants (Fig. 1D).

### Example 4 - slhak5 plants show reduced Cs+ uptake and accumulation

In order to clarify the contribution of *slhak5* in Cs+ uptake and accumulation, K+-starved plants were assayed for Cs+ uptake from a 0.02 mM solution. The results obtained clearly showed that Cs+ uptake rates were lower in slhak5-1 plants in comparison to those of WT plants (1.29 ± 0.72 µmol Cs+ g⁻¹_{rootDW}h⁻¹ vs 7.52 ± 0.72 µmol Cs+ g⁻¹_{rootDW}h⁻¹, respectively) (Fig. 2).

Another experiment was designed to study Cs+ accumulation in different plants organs and importantly in fruits. Plants were grown for 7d in control solution containing 1.4 mM K+ and then transferred to a solution containing 0.3 mM K+ and 0.02 mM Cs+ for 90 d. Plant material was harvested and the internal Cs+ and K+ concentrations determined. It was observed that *slhak5-1* plants accumulated much less Cs+ in their organs than WT plans (Fig. 3A). The Cs+ concentrations were 61-, 52-, 18-, 7- and 22-fold higher in WT roots, stems, young leaves, mature leaves and fruits respectively than in *slhak5-1* ones (Fig. 3A). The K+ concentrations of plant organs did not show differences between the two lines, except in fruits (Fig. 3B). Therefore, the K+/Cs+ ratios were much higher in slhak5-1 than in WT plants, indicating a higher discrimination between K+ and Cs+ in plants lacking *slhak5* (Fig. 3C). The Cs+ transfer factors (TF_{fruit}) from the external solution to the fruit were calculated and important differences between WT and mutant plans were observed. While WT plants showed a TF_{fruit} of 151.6 ± 28, *slhak5-1* plants showed a TF_{fruit} of 8.5 ± 0.7. It is worth to mention that the lower TF_{fruit} of *slhak5-1* plants was achieved without affecting tissue K+ content.

### Example 5 - slhak5 plants show reduced fruit and seed production.

Plants were grown in K+ sufficient (1.4 mM K+) nutrient solution until fructification. Tomato fruits of the transgenic plant of the invention, were harvested at different time points as they ripen. Fruit production was delayed in *slhak5-1* in relation to WT plants (Fig. 4A). At the date of the first fruit harvest in WT plants no tomato fruits could be collected in the *slhak5-1* line. *slhak5-1* plants showed a much lower total fruit production than WT plants and whereas the former line produced an average of 22.6 ± 3.0 g of tomatoes plant⁻¹ the latter one produced 51.6 ± 9.7 g of tomatoes plant⁻¹ (Fig. 4B). In addition, the number of seeds per tomato fruit was much smaller in *slhak5-1* plants, with 1.5 ± 1.5 seeds tomato⁻¹ in comparison with the 15.6 ± 4.8 seeds tomato⁻¹ in WT plants (Fig. 4C).

## Claims

1. A transgenic plant, a reproductive or propagating plant material, or a cultured plant cell **characterized in that** it comprises a gene with at least 90%, 95% or 100% sequence identity with SEQ ID NO: 2 wherein said gene has been silenced.

2. The transgenic plant, the reproductive or propagating plant material, or the cultured plant cell according to claim 1 **characterized in that** it comprises the polypeptide according to SEQ ID NO: 1.

3. The transgenic plant, the reproductive or propagating plant material, or the cultured plant cell according to any of claims 1 to 2 belonging to *Solanum sp.* or *Capsicum sp.* genera.

4. The transgenic plant, the reproductive or propagating plant material, or the cultured plant cell according to claim 3 belonging to a species selected from the list consisting of: *Solanum lycopersicum, S. tuberosum, S. pennellii, S. pimpinellifolium, S. peruvianum, S. cheesmanii, S. galapagense, S. chilense, S. melongena, S. aethiopicum, S. quitoense, S. torvum, S. muricatum, S. betaceum and Capsicum annuum.*

5. A cell, silique, seed, progeny or part of the transgenic plant according to any of claims 1 to 4, **characterized in that** it comprises a gene with at least 90%, 95% or 100% sequence identity with SEQ ID NO: 2 wherein said gene has been silenced.

6. The cell, silique, seed, progeny or part of the transgenic plant according to claim 5, **characterized in that** it comprises the polypeptide according to SEQ ID NO: 1.

7. The cell, silique, seed, progeny or part of the transgenic plant according to any of claims 5 to 6, wherein the part of the plant is selected from the list consisting of: a leaf, a stem, a flower, an ovary, a fruit or a callus.

8. A method for producing a transgenic plant, a reproductive or propagating plant material, or a cultured plant cell according to any of claims 1 to 4, or a cell, silique, seed, progeny or part of the transgenic plant according to any of claims 5 to 7, said method comprising the silencing of a gene with at least 90%, 95% or 100% sequence identity with SEQ ID NO: 2.

9. A use of the transgenic plant, reproductive or propagating plant material, cultured plant cell according to any of claims 1 to 4, and/or of the cells, siliques, seeds, progeny or part of the transgenic plant according to claims 5 to 7, for producing an agro-industrial product, preferably wherein the agro-industrial product is a food or a feed.

10. An agro-industrial product production method, preferably wherein the agro-industrial product is a food or a feed, comprising:
a) culturing the transgenic plant, reproductive or propagating plant material, plant cell, according to any of claims 1 to 4, or cell, silique, progeny or part of the transgenic plant according to any of claims 5 to 7, and
b) preparing the agro-industrial product.

11. A method for producing a transgenic plant with low intake of Cesium compared to a wild-type control plant, preferably wherein the Cesium is Radiocesium, and that is parthenocarpy, said method comprising culturing the transgenic plant, reproductive or propagating plant material, plant cell, according to any of claims 1 to 4, or cell, silique, progeny or part of the transgenic plant according to any of claims 5 to 7 in a culturing medium comprising a concentration of potassium of more than 100 µM.
